# FASCICULE DE BREVET EUROPEEN

(11) **EP 1 511 527 B1**
(45) Date de publication et mention de la délivrance du brevet: **19.12.2012**
(21) Numéro de dépôt: 03752824.7
(22) Date de dépôt: 16.05.2003
(51) Int. Cl.: A61M 5/158

(54) **DISPOSITIF MEDICAL DEFORMABLE POUR LE RETRAIT D'UNE AIGUILLE**
VERFORMBARE MEDIZINISCHE VORRICHTUNG ZUR ENTFERNUNG EINER NADEL
DEFORMABLE MEDICAL DEVICE FOR REMOVING A NEEDLE

(30) Priorité: 17.05.2002 FR 0206091
(43) Date de publication de la demande: 09.03.2005
(73) Titulaire: Districlass Medical S.A., 42000 Saint-Etienne (FR)
(72) Inventeur: BERTHEAS, Jacques, F-69380 Lissieu (FR)
(74) Mandataire: Schmitt, John
(86) Numéro de dépôt international: PCT/FR2003/001490
(87) Numéro de publication internationale: WO 2003/097119

(56) Documents cités:
- EP-A- 0 821 974
- WO-A-98/34675
- US-A- 4 632 671
- US-A- 4 986 819
- US-A- 5 224 936

## Description

Le présente invention est relative à un dispositif médical déformable élastiquement sous l'effet d'un liquide ou de l'air sous pression pour permettre le retrait d'une aiguille et plus particulièrement d'une aiguille de Huber.

Il a été constaté que, pour réaliser le retrait d'une aiguille implantée, le personnel soignant fournit un effort de traction qui provoque immédiatement après l'extraction de l'aiguille un rebond entraînant un risque de piqûre des doigts du personnel soignant se trouvant à proximité de l'aiguille.

Un dispositif de protection des doigts du personnel ou de l'outilisateur est décrit dans le document US 5224936 enseignant une seringue hypodermique comprenant une enveloppe gonflable de protection d'aiguille raccordée à une tubulure.

Ainsi, le dispositif médical suivant la présente invention permet de réaliser autour de l'aiguille une enveloppe gonflable permettant la protection des doigts du personnel et le retrait progressif et sans effort de la dite aiguille d'une chambre implantable, par exemple.

Egalement, le dispositif médical suivant la présente invention permet d'assurer un confort pour le malade par une enveloppe sous aiguille, pendant toute la durée de l'implantation.

Le dispositif médical suivant la présente invention comporte une enveloppe gonflable raccordée à une tubulure, ladite enveloppe comportant au moins un espace de réception pour le passage d'une canule.

Le dispositif médical suivant la présente invention comporte une enveloppe gonflable raccordée à une tubulure et un disque rigide formant un plateau horizontal disposé et fixé au-dessus de l'enveloppe.

Le dispositif médical suivant la présente invention comporte une enveloppe gonflable et un disque rigide comportant respectivement des espaces de réception pour le passage de la canule.

Le dispositif médical suivant la présente invention comporte une enveloppe présentant, à partir de son milieu et en direction de l'extérieur, un espace de réception constitué d'une fente formant deux extrémités distinctes et étanches, afin que ladite enveloppe présente un profil en forme de C.

Le dispositif médical suivant la présente invention comporte une enveloppe présentant un espace de réception de forme annulaire.

Le dispositif médical suivant la présente invention comporte un disque rigide comprenant, à partir de son centre, une fente présentant un profil en forme de V dont la base la plus ouverte est disposée du coté de la périphérie dudit disque.

Le dispositif médical suivant la présente invention comporte un disque rigide fixé dans la partie supérieure de l'enveloppe de manière que son centre soit confondu avec celui de ladite l'enveloppe.

Le dispositif médical suivant la présente invention comporte un disque rigide solidaire d'un corps raccordé à une canule effilée traversant ledit disque afin de coopérer à l'intérieur d'un l'espace libre ménagé dans l'enveloppe.

Le dispositif médical suivant la présente invention comporte un raccord comprenant, perpendiculairement à la canule effilée, une tubulure solidaire à son extrémité libre d'une valve anti-retour.

La description qui va suivre en regard des dessins annexés, donnés à titre d'exemples non limitatifs, permettra de mieux comprendre l'invention, les caractéristiques qu'elle présente et les avantages qu'elle est susceptible de procurer:
Figure 1 est une vue de coté montrant le dispositif médical suivant la présente invention en position de repos.
Figure 2 est une vue de coté illustrant le dispositif médical suivant la présente invention en position gonflée.
Figure 3 est une vue de dessus représentant le dispositif médical suivant la présente invention en position gonflée.
Figures 4 à 9 sont des vues montrant les différentes étapes d'utilisation du dispositif médical de figures 1 à 3.
Figure 10 est une vue de coté montrant une variante du dispositif médical suivant la présente invention en position de repos.
Figures 11 et 12 sont des vues illustrant le dispositif médical de figure 10 suivant la présente invention en position gonflée.
Figures 13 à 15 sont des vues représentant les différentes étapes d'utilisation de la variante du dispositif médical de figures 10 à 12.

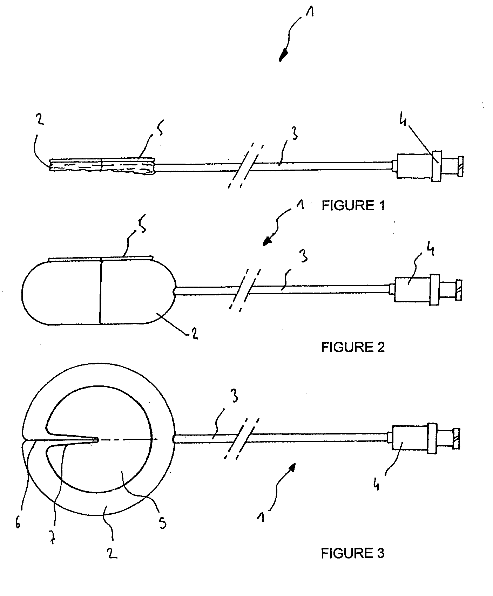

On a montré en figures 1 à 3 un dispositif médical 1 comportant une enveloppe 2 étanche et déformable sous l'effet d'une pression extérieure et une tubulure 3 solidaire d'une valve anti-retour 4.

La tubulure 3 communique de manière étanche, à l'opposé de la valve anti-retour 4, à l'intérieur de l'enveloppe 2 pour pouvoir gonfler cette dernière au moyen d'un liquide ou de l'air sous pression.

Le dispositif médical 1 peut comporter au-dessus de l'enveloppe 2 un disque rigide 5 formant un plateau horizontal permettant d'assurer une répartition des efforts de pression lors du gonflage de ladite chambre.

L'enveloppe 2 comporte, à partir de son milieu et en direction de l'extérieur, une fente 6 formant deux extrémités distinctes et étanches afin que ladite enveloppe présente un profil en forme de C.

Le disque rigide 5 est fixé sur l'enveloppe 2 de manière que son centre soit confondu avec celui de ladite chambre.

Le disque rigide 5 comporte à partir de son centre une fente 7 présentant un profil en forme de V dont la base la plus ouverte est disposée du coté de la périphérie dudit disque.

La fente 7 du disque rigide 5 est prévue au-dessus de celle 6 de l'enveloppe 2 du dispositif médical 1.

On note que la tubulure 3 est portée, par exemple, sur le même axe que celui des fentes 6, 7 de l'enveloppe 2 et du disque rigide 5.

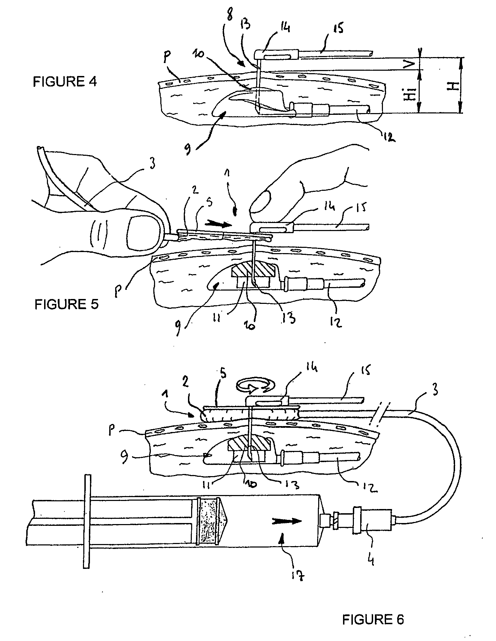

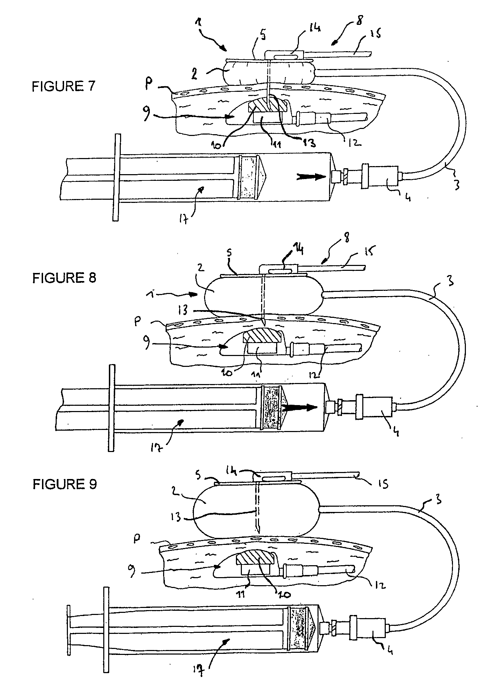

On a représenté en figures 4 à 9 les différentes étapes d'utilisation du dispositif médical 1 suivant la présente invention pour le retrait d'une aiguille de Huber 8 d'une chambre implantable 9, par exemple.

La chambre 9 est implantée sous la peau **P** d'un patient pour le traitement, par exemple, de chimiothérapie.

La chambre 9 est reliée à l'extérieur, par l'intermédiaire de l'aiguille de Huber 8 qui traverse une membrane 10 pour venir déboucher à l'intérieur d'un logement 11.

Ce dernier est relié par l'intermédiaire d'un cathéter 12 à un vaisseau du patient pour permettre l'injection de produits de traitement.

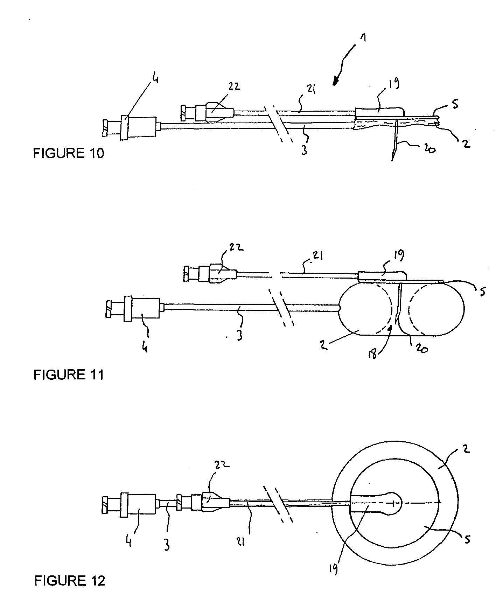

L'aiguille de Huber 8 comporte une canule effilée 13 autour de laquelle est surmoulée, à l'opposé de la pointe et suivant une direction perpendiculaire à l'axe de ladite canule, un corps 14 de forme quelconque qui est raccordé de manière étanche à une tubulure 15 solidaire d'un système de connexion semblable, par exemple, à celui 22 (figure 10)

Lorsque l'aiguille de Huber 8 est introduite à l'intérieur de la chambre 9, on note que :

| | |
|---|---|
| ❖ | la hauteur comprise entre la face inférieure du corps 14 et la pointe effilée de la canule 13 est **H**, |
| ❖ | la hauteur d'implantation de l'aiguille sous la peau **P**, jusqu'au fond du logement 11 de la chambre 9 est **Hi**, |
| ❖ | la hauteur de l'espace entre la face externe de la peau **P** et la face inférieure du corps 14 est **V**. |

### Mode d'utilisation

Le dispositif médical 1 et plus particulièrement de l'enveloppe 2 solidaire du disque rigide 5 est placé dans l'espace **V** prévu entre la peau **P** du patient et la face inférieure du corps 14 de l'aiguille de Huber 8.

Le dispositif médical 1 est introduit par une voie d'accès qui se trouve, par exemple, à l'opposé de la tubulure 15 de l'aiguille de Huber 8 ou par une autre voie qui ne se trouve pas dans l'axe de ladite tubulure 15.

Le dispositif médical 1 est positionné dans l'espace **V** de manière que la canule 13 de l'aiguille de Huber 8 coopère avec les fentes 6 et 7 de l'enveloppe 2 et du disque 5.

Le dispositif médical 1 est correctement positionné lorsque la canule 13 est placée dans le fond de chaque fente 6, 7, c'est à dire, lorsque la canule 13 est portée par l'axe vertical et central de l'enveloppe 2 et du disque 5.

Ensuite, le dispositif médical 1 est entraîné en rotation autour de la canule 13 de l'aiguille de Huber 8 pour orienter la tubulure 3 dudit dispositif du coté de la tubulure 15 de ladite aiguille pour améliorer le confort du patient.

Le dispositif médical 1 et plus particulièrement l'enveloppe 2 est gonflée au moyen d'un liquide ou de l'air qui est injecté par l'intermédiaire d'une seringue 17 connectée sur la valve anti-retour 4 solidaire de la tubulure 3.

Le volume de l'enveloppe 2 est gonflé pour en premier lieu compléter l'espace **V** et ensuite augmenter progressivement pour retirer la canule 13 de l'aiguille de Huber 8 de la membrane 10 de la chambre implantable 9 et de la peau **P** du patient.

Ainsi, le dispositif médical 1 permet le retrait progressif, grâce au déplacement vertical du disque 5 sous l'augmentation du volume de l'enveloppe 2, de l'aiguille de Huber 8 sans risque du phénomène de rebond et donc sans risque que le personnel soignant soit piqué ou blessé.

On note également que l'enveloppe 2 du dispositif médical 1 forme un élément protecteur autour de la canule 13 de l'aiguille de Huber 8 et limite le risque de piqûre pour le personnel soignant.

On a illustré en figures 10 à 12 une variante du dispositif médical 1 qui comporte une enveloppe 2 déformable sous l'effet d'une pression extérieure, une tubulure 3 solidaire d'une valve anti-retour 4 et une canule effilée 20 formant une aiguille.

La tubulure 3 communique de manière étanche, à l'opposé de la valve anti-retour 4, à l'intérieur de l'enveloppe 2 pour pouvoir gonfler cette dernière au moyen d'un liquide ou de l'air sous pression.

Le dispositif médical 1 comporte au-dessus de l'enveloppe 2 un disque rigide 5 formant un plateau horizontal permettant d'assurer une répartition des efforts de pression lors du gonflage de ladite enveloppe 2.

L'enveloppe 2 présente en position gonflée un volume défini de forme annulaire comportant en son milieu un espace libre 18 traversant verticalement le dispositif médical 1.

Le disque rigide 5 est fixé dans la partie supérieure de l'enveloppe 2 de manière que son centre soit confondu avec celui de ladite enveloppe 2. Le disque 5 est solidaire à l'opposé de l'enveloppe 2 d'un corps 19 raccordé à une canule effilée 20 traversant ledit disque afin de coopérer à l'intérieur de l'espace libre 18 de l'enveloppe 2.

Le corps 19 comporte perpendiculairement à la canule 20 une tubulure 21 solidaire à son extrémité libre d'un système de connexion 22. La tubulure 21 est disposée au-dessus et dans un plan parallèle à celui contenant la tubulure 3 pour l'alimentation de l'enveloppe 2.

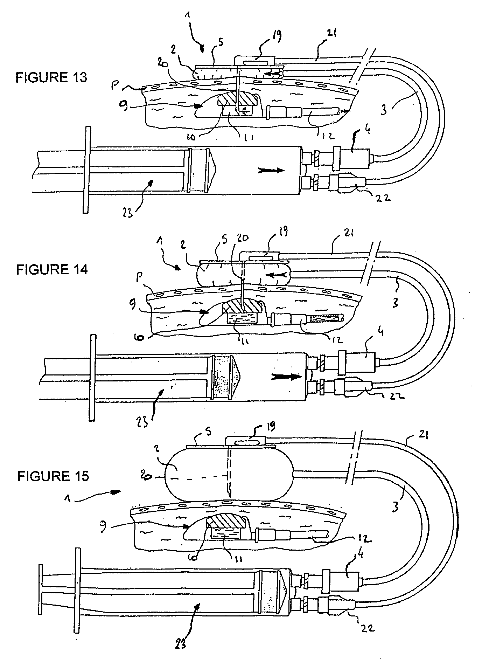

On a montré en figures 13 à 15 les différentes étapes d'utilisation du dispositif médical 1 suivant la présente invention pour l'implantation et le retrait de la canule effilée 20 d'une chambre implantable 9, par exemple.

La chambre 9 est implantée sous la peau **P** d'un patient pour le traitement, par exemple, de chimiothérapie.

La chambre 9 est reliée à l'extérieur, par l'intermédiaire de la canule 20 qui traverse une membrane 10 pour venir déboucher à l'intérieur d'un logement 11.

Ce dernier est relié par l'intermédiaire d'un cathéter 12 à un vaisseau du patient pour permettre l'injection de produits de traitement.

Le dispositif médical 1 est mis en place de manière que sa canule 20 débouche à l'intérieur du logement 11 de la chambre implantable 9 se trouvant sous la peau **P.**

L'enveloppe 2 en position dégonflée du dispositif médical 1 vient en appui contre la face externe de la peau **P** du patient.

Ainsi, le personnel soignant peut introduire, au travers de la tubulure 21 de la canule 20, un produit de traitement à l'intérieur du logement 11 de la chambre 9 afin que ce dernier puisse se diffuser au moyen du cathéter 12 raccordé à une artère dans le sang du patient.

Après introduction du produit de traitement, le personnel soignant peut procéder au nettoyage du logement 11 de la chambre implantable 9 en injectant, par exemple, du sérum physiologique au moyen d'une seringue 23 raccordée au système de connexion 22.

Le personnel soignant peut simultanément à l'aide de la même seringue 23 ou d'une autre distincte ou d'une seringue avec un raccord en y raccordée à la valve anti-retour 4, injecter le même liquide à l'intérieur de l'enveloppe 2 pour commencer son gonflage.

L'injection simultanée de sérum physiologique à l'intérieur du logement 11 et de l'enveloppe 2 permet le rinçage et le verrou positif de la chambre implantable 9 et de son cathéter 12, ainsi que le gonflage de l'enveloppe 2.

Le verrou positif de la chambre 9 étant assuré, le volume de l'enveloppe 2 est gonflé progressivement pour retirer la canule 20 de la membrane 10 de la chambre implantable 9 et éventuellement de la peau **P** du patient.

Ainsi, le dispositif médical 1 permet l'introduction de produit traitant et le retrait progressif, grâce au déplacement vertical du disque 5 sous l'augmentation du volume de l'enveloppe 2, sans risque du phénomène de rebond et donc sans risque que le personnel soignant soit piqué ou blessé.

On note également que l'enveloppe 2 du dispositif médical 1 forme un élément protecteur autour de la canule 20 et limite tout risque de piqûre pour le personnel soignant.

On constate que le dispositif médical 1 suivant la présente invention permet par l'intermédiaire de son enveloppe gonflable 2 de décoller et de retirer la canule 13, 20 de la peau.

Il doit d'ailleurs être entendu que la description qui précède n'a été donnée qu'à titre d'exemple et qu'elle ne limite nullement le domaine de l'invention dont on ne sortirait pas en remplaçant les détails d'exécutions décrits par tout autre équivalent.

## Revendications

1. Dispositif médical pour le retrait d'une canule effilée (13, 20) formant une aiguille préalablement implantée, par exemple, dans une chambre implantable (9), **caractérisé en ce qu'**il comporte une enveloppe gonflable (2) raccordée à une tubulure (3) solidaire d'une valve anti-retour (4), un disque rigide (5) formant un plateau horizontal au-dessus de l'enveloppe (2) et des espaces de réception (6, 7 ;18) ménagés dans l'enveloppe (2) et le disque (5) pour le passage de la canule (13, 20), lesdits espaces de réception de l'enveloppe (2) présentant un profil en forme de fente (6) ou annulaire (18).

2. Dispositif médical suivant la revendication 1, **caractérisé en ce que** l'enveloppe (2) comporte, à partir de son milieu et en direction de l'extérieur, un espace de réception constitué d'une fente (6) formant deux extrémités distinctes et étanches afin que ladite enveloppe présente un profil en forme de C.

3. Dispositif médical suivant la revendication 1, **caractérisé en ce que** l'enveloppe (2) présente un espace de réception constitué d'une forme annulaire comportant en son milieu un espace libre (18).

4. Dispositif médical suivant la revendication 1, **caractérisé en ce que** le disque rigide (5) comporte à partir de son centre un espace de réception constitué d'une fente (7) présentant un profil en forme de V dont la base la plus ouverte est disposée du coté de la périphérie dudit disque.

5. Dispositif médical suivant la revendication 1, **caractérisé en ce que** le disque rigide (5) est fixé dans la partie supérieure de l'enveloppe (2) de manière que son centre soit confondu avec celui de ladite l'enveloppe (2).

6. Dispositif médical suivant la revendication 3, **caractérisé en ce que** le disque (5) est solidaire d'un corps (19) raccordé à une canule effilée (20) traversant ledit disque afin de coopérer à l'intérieur d'un espace de réception (18) ménagé dans l'enveloppe (2)

7. Dispositif médical suivant la revendication 6, **caractérisé en ce que** le corps (19) comporte perpendiculairement à la canule (20) une tubulure (21) solidaire à son extrémité libre d'un système de connexion (22).

8. Dispositif médical suivant la revendication 7, **caractérisé en ce que** la tubulure (21) est disposée au-dessus et dans un plan parallèle à celui contenant la tubulure (3) pour l'alimentation de l'enveloppe (2).

## Claims

1. A medical device for removing a tapered cannula (13, 20) forming a needle previously implanted, for example, in an implanted port (9), **characterized in that** it comprises an inflatable sheath (2) connected to a tubing (3) affixed to a non-return valve (4), a rigid disk (5) forming a horizontal platform above the sheath (2) and receiving spaces (6, 7, 18) arranged in the sheath (2) and the disk (5) for the passage of the cannula (13, 20), said receiving spaces of the sheath (2) having a slit (6) or annular (18) shaped profile.

2. Medical device according to claim 1, **characterized in that the** sheath (2) comprises, starting from its center and extending toward the outside, a receiving space formed by a slit (6) creating two separate and leaktight ends, so that said sheath has a C-shaped profile.

3. Medical device according to claim 1, **characterized in that the** sheath (2) has a receiving space constituted of an annular shape comprising, in its center, a free space (18).

4. Medical device according to claim 1, **characterized in that the** rigid disk (5) comprises, from its center, a receiving space constituted of a slit (7) having a V-shaped profile, the most open base of which is arranged on the side of the periphery of said disk.

5. Medical device according to claim 1, **characterized in that the** rigid disk (5) is fixed to the upper portion of the sheath (2) so that its center is one with that of said sheath (2).

6. Medical device according to claim 3, **characterized in that the** disk (5) is affixed to a body (19) connected to a tapered cannula (20) which extends through said disk so as to cooperate inside a receiving space (18) housed in the sheath (2).

7. Medical device according to claim 6, **characterized in that the** body (19) comprises, perpendicularly to the cannula (20), a tubing (21) affixed at its free end to a connection system (22).

8. Medical device according to claim 7, **characterized in that** the tubing (21) is arranged above and in a plane parallel to that containing the tubing (3) for supplying the sheath (2).

## Patentansprüche

1. Medizinische Vorrichtung zum Zurückziehen einer spitzen Kanüle (13, 20), die eine Nadel bildet, die beispielsweise zuvor in eine implantierbare Kammer (9) implantiert wurde, **dadurch gekennzeichnet, dass** die Vorrichtung eine aufblasbare Hülle (2), die mit einer Leitung (3) verbunden ist, die fest mit einem Rückschlagventil (4) verbunden ist, eine starre Scheibe (5), die über der Hülle (2) eine horizontale Platte bildet, und Aufnahmeräume (6, 7; 18), die in der Hülle (2) und der Scheibe (5) zum Durchtritt der Kanüle (13, 20) eingerichtet sind, umfasst, wobei die Aufnahmeräume der Hülle (2) ein Profil in Schlitzform (6) oder Ringform (18) aufweisen.

2. Medizinische Vorrichtung nach Anspruch 1, **dadurch gekennzeichnet, dass** die Hülle (2) ab ihrer Mitte und in Richtung der Außenseite einen Aufnahmeraum umfasst, der von einem Schlitz (6) gebildet wird, der zwei verschiedene und dichte Enden bildet, so dass die Hülle ein Profil in C-Form aufweist.

3. Medizinische Vorrichtung nach Anspruch 1, **dadurch gekennzeichnet, dass** die Hülle (2) einen Aufnahmeraum aufweist, der von einer Ringform gebildet wird, die in ihrer Mitte einen freien Raum (18) umfasst.

4. Medizinische Vorrichtung nach Anspruch 1, **dadurch gekennzeichnet, dass** die starre Scheibe (5) ab ihrem Zentrum einen Aufnahmeraum umfasst, der von einem Schlitz (7) gebildet wird, der ein Profil in V-Form aufweist, dessen am meisten geöffnete Basis an der Seite des Umfangs der Scheibe angeordnet ist.

5. Medizinische Vorrichtung nach Anspruch 1, **dadurch gekennzeichnet, dass** die starre Scheibe (5) derart in dem oberen Teil der Hülle (2) befestigt ist, dass ihr Zentrum mit dem der Hülle (2) zusammenfällt.

6. Medizinische Vorrichtung nach Anspruch 3, **dadurch gekennzeichnet, dass** die Scheibe (5) fest mit einem Körper (19) verbunden ist, der mit einer spitzen Kanüle (20) verbunden ist, die durch die Scheibe hindurchgeht, um mit dem Inneren eines Aufnahmeraums (18) zusammenzuwirken, der in der Hülle (2) eingerichtet ist.

7. Medizinische Vorrichtung nach Anspruch 6, **dadurch gekennzeichnet, dass** der Körper (19) senkrecht zu der Kanüle (20) eine Leitung (21) umfasst, die an ihrem freien Ende fest mit einem Verbindungssystem (22) verbunden ist.

8. Medizinische Vorrichtung nach Anspruch 7, **dadurch gekennzeichnet, dass** die Leitung (21) über und in einer Ebene parallel zu der Ebene, die die Leitung (3) enthält, zur Versorgung der Hülle (2) angeordnet ist.
